# EUROPEAN PATENT APPLICATION

(11) **EP 4 571 779 A1**
(43) Date of publication of application: **18.06.2025**
(21) Application number: 23216521.7
(22) Date of filing: 14.12.2023
(51) Int. Cl.: G16H 50/30

(54) **GESTATIONAL WEIGHT GAIN PREDICTION**

(71) Applicant: Koninklijke Philips N.V., 5656 AG Eindhoven (NL)
(72) Inventor: JOHNSON, Mark Thomas, Eindhoven (NL); TEN KATE, Warner Rudolph Theophile, Eindhoven (NL); BONOMI, Alberto Giovanni, Eindhoven (NL); SARTOR, Francesco, Eindhoven (NL); PALERO, Jonathan Alambra, Eindhoven (NL); KOOIJMAN, Gerben, Eindhoven (NL)
(74) Representative: Philips Intellectual Property & Standards

(57) **Abstract**

Disclosed concepts aim to provide schemes, solutions, concepts, designs, methods, and systems pertaining to improved prediction of gestational weight gain (GWG). Specifically, a data series describing weight of the pregnant subject at least twice per day (e.g., after waking up, and before going to sleep) during an early stage of pregnancy (e.g., during the first 30 days of pregnancy, or during the first trimester) is used to determine a historic GWG curve of the subject. As energy balance in pregnancy is defined as energy intake equal to energy expenditure plus dynamic energy storage, a specific GWG prediction model is used to generate the historic GWG curve, rather than a generic weight gain model. From the historic GWG curve, a future GWG value of the subject may be predicted. This future GWG value may be used to assess a pregnancy risk score, and/or to generate lifestyle recommendations for the subject.

## Description

### FIELD OF THE INVENTION

The present invention relates to the field of predicting gestational weight gain of a subject.

### BACKGROUND OF THE INVENTION

The maintenance of a particular body weight is difficult in modern society, partially due to easy access to high caloric food. Generally, when calorie intake exceeds calorie expenditure body weight increases. This may result, over time, in causing the person to be overweight or even obese. In addition, the lack of proper nutrition and weight loss can be a serious issue in the elderly population, whose biological signalling of hunger and satiety may be inadequate.

Another population category in need of managing their weight are those who are pregnant. For optimal pregnancy outcomes, the pregnant individual needs to accumulate an appropriate level of fat, which depends on the individual's body shape. Indeed, an individual with low body weight will require more fat mass accumulation than an overweight individual. Given the high energy density of fat mass, these differences greatly affect energy intake requirements. In contrast, the energy stored in foetal and placental tissues (depending on the size of the fetus) is comparable between most individuals, and has relatively small impact on energy intake requirements.

Accordingly, there exists a need to help pregnant individuals manage their gestational weight gain (GWG). Indeed, projection of GWG would enable the generation of a prediction of a pregnancy risk level, which may be used to inform medical device and (potential) future medical interventions. Further, such a prediction may form the basis for improving adherence to a recommended lifestyle behavioural change during pregnancy.

### SUMMARY OF THE INVENTION

The invention is defined by the claims.

According to examples in accordance with an aspect of the invention, there is provided a method for predicting future gestational weight gain (GWG) of a subject, comprising:
obtaining a data series of weight values describing weight of the subject at least twice per day during an early stage of pregnancy of the subject and a respective gestational age of the subject;
processing the data series, with a GWG prediction model, to determine a historic GWG curve of the subject describing GWG of the subject over the early stage of pregnancy; and
predicting a future GWG value of the subject based on the historic GWG curve.

The disclosed invention aims to provide an improved prediction of GWG. Specifically, a data series describing weight of the pregnant subject at least twice per day (e.g., after waking up, and before going to sleep) during an early stage of pregnancy (e.g., during the first 30 days of pregnancy, or during the first trimester) is used to determine a historic GWG curve of the subject. As energy balance in pregnancy is defined as energy intake equal to energy expenditure plus dynamic energy storage, a specific GWG prediction model is used to generate the historic GWG curve, rather than a generic weight gain model. From the historic GWG curve, a future GWG value of the subject may be predicted. This future GWG value may be used to assess a pregnancy risk score, and/or to generate lifestyle recommendations for the subject.

Repeated micro-weighing (i.e. measuring weight of a subject more than once per day) is a method to accurately track weight gain of a subject. However, for pregnant individuals, predicting future weight gain based on historic weight gain is not straightforward, as the fetus grows in a non-linear manner (i.e., rate of growth and therefore contribution to weight gain depends on the stage of pregnancy/gestational age). Therefore, to gather an accurate understand the historic weight gain of the subject (i.e., what weight gain is attributable to fetus weight gain, and what weight gain is attributable to fat storage), as well as to predict any future gestational weight, the respective gestational age of the subject at the time that weight measurements are taken should be tracked.

Put another way, it has been realised that by measuring weight at least twice per day (preferably before and after sleep), daily calorie intake, expenditure, nutritional intake and calorie storage can be derived. This has not been applied to pregnant subjects. For pregnant subjects, there is an added layer of complexity in that some energy is used to develop the baby (and so needs to be added to the model as energy usage that does not result in weight loss), which will depend on the gestational age of the subject.

Thus, embodiments of the invention determine a historical GWG curve from weight data taken at least twice per day in early pregnancy (i.e. micro-weight tracking data). This GWG curve/data can then be extrapolated to predict GWG in later stages of pregnancy. Once that is known, recommendations can be generated based on comparing the predicted GWG to a recommended/ideal GWG. In addition, the specific determined calorie intake, expenditure, nutritional intake and calorie storage can be used for various aspects of recommendations, as well as to improve the historic GWG curve, and predicted future GWG value.

Disclosed embodiments of the invention take into account the gestational age of the subject when processing the data series of measured weights using the GWG prediction model to generate an accurate GWG curve. A resulting future GWG value derived from the GWG curve may therefore be more accurate, and so more appropriate advice and actions may be taken.

To clarify, the gestational age of the subject describes the how far along the pregnancy the subject is. Technically, the gestational age may have different measures, such as a length of time since the last menstrual period of the subject, or via another method. The data series comprises a set of weight values of the subject, with each weight value having an associated gestational age of the subject when the measurement was taken.

The early stage of the pregnancy may be considered the first 100 days of the pregnancy, or may be the first trimester of the pregnancy. In some embodiments, the weight values may span only the first 30 days of pregnancy. During this early stage of pregnancy, the daily or even weekly weight change of the subject is small, and therefore normal fluctuations of body weight may greatly impact the accuracy of the determined GWG curve. As a result, the weight of the subject must be measured at least twice per day such that the GWG curve is accurate.

More specifically, the data series of weight values may describe the weight of the subject at least each day before sleep and after sleep.

It has recently been noted that weight loss during sleep correlates with total energy intake. In addition, weight loss during sleep also correlates with individual nutritional intake, such as carbohydrate intake, fat intake, and protein intake. In other words, a person with a greater weight loss rate during the night/during sleep generally will have consumed a larger number of calories, carbohydrates, proteins and fats than a person with a lower weight loss rate. This may be explained by a variation in the resting metabolic rate of the individual consuming a greater number of calories, fats, carbohydrates or proteins.

Thus, by ensuring the data series of weight values contains this information, a more accurate GWG curve can be determined. In turn, a resultant future GWG prediction may be more accurate. That is, weight values taken shortly before sleep and shortly after sleep each day may be used to improve accuracy of the determined GWG curve.

Alternatively, the data series of weight values may describe the weight of the subject at least once per hour.

The more frequent the weight value data is gathered, the less susceptible to random alterations (e.g., variations responsive to normal actions of the subject) the historic GWG curve will be. This is because these variations can be distinguished and accounted for by the GWG prediction model when given a data series of weight values with a sufficient resolution.

In addition, the data series of weight values may further describe the weight of the subject at least twice per day during a time period preceding pregnancy.

By also including weight values from a time period before pregnancy, lifestyle trends and changes of the subject (that may not be directly attributable to pregnancy) may be identified and appreciated. Accordingly, a more accurate GWG curve may be generated.

In some embodiments, the method may further comprise generating at least one of current calorie intake data, current calorie expenditure data, current calorie storage data or current nutritional intake data based on the data series and a current gestational age of the subject.

As stated above, repeated micro-weighing (i.e. measuring weight of a subject more than once per day) weight loss between two points in time within a day of each other (e.g., one before and one after sleep) correlates with individual nutritional intake, such as carbohydrate intake, fat intake, and protein intake. Accordingly, this information may be generated from the data series.

In this case, the historic GWG curve of the subject may further comprise processing the at least one of the current calorie intake data, current calorie expenditure data, current calorie storage data or current nutritional intake data with the GWG prediction model.

Such information may be useful to separately generate and then provide to the GWG prediction model. This may ensure such factors are taken into account when generating the historic GWG curve, improving accuracy of the historic GWG curve.

In addition, the method may further comprise generating a recommendation describing a recommended change to a lifestyle of the subject based on the future GWG value, and at least one of the current calorie intake data, current calorie expenditure data, current calorie storage data or current nutritional intake data.

This information may be leveraged to provide a personalized and useful recommendation regarding a lifestyle change of the subject that may lead to (an increased chance of) improved pregnancy outcomes.

The recommendation may comprise a calorie intake recommendation, a nutritional intake recommendation, or an exercise recommendation.

For example, the recommendation may encourage the subject to consume more or less food, and more or less of specific foods. In addition, the recommendation may encourage the subject to exercise more or less.

When the recommendation comprises an exercise recommendation, the method may further comprise adjusting the exercise recommendation based on gait information describing a gait stability of the subject. Accordingly, a suitability of the recommendation may be improved.

Furthermore, the recommendation may be based on a target (growth) curve. This target curve may be set by a physician/medical professional. Of course, the target curve may also be adjusted as the pregnancy progresses.

In some embodiments, the method may further comprise modifying the data series based on subject context information describing at least one of movement, temperature, water loss, cardiovascular activity (i.e., a photoplethysmography signal), or resting metabolic rate of the subject during the early stage of pregnancy of the subject.

In this way, variations due to changes in movement, temperature, water retention/loss, cardiovascular activity (indicative of stress), and changes in resting metabolic rate may be removed from the data series. As a result, a historic GWG curve obtained from the GWG prediction model responsive to imputing the data series may be improved.

That is, it is not always possible for the subject to repeat the exact conditions of weight measurement each time. Indeed, some factors may be entirely outside of control by the subject. Therefore, accounting for these factors may improve accuracy of the data series. The subject context information may be measured, acquired from observations, or deduced from time and location context based information.

Further embodiments may include additional steps of obtaining weight-related physiological data describing at least one physiological parameter of the subject; and adjusting the predicted future GWG value based on the obtained weight-related physiological data.

In particular, the at least one physiological parameter may comprise at least one of a pre-pregnancy weight, a pre-pregnancy BMI, an age, a height, an ethnicity, a stress value a pre-pregnancy lifestyle parameter value, or a pregnancy lifestyle parameter value.

Each of these physiological parameters may impact the general trend of the GWG of the subject. Thus, taking these into account when extrapolation the historic GWG curve to determine the predicted future GWG value may improve an accuracy of said value.

In some example embodiments, processing the data series to determine the historic GWG curve may comprise processing the data series with a regression analysis model, and wherein predicting the future GWG value of the subject comprises extrapolating the historic GWG curve.

The method may further comprise generating a pregnancy risk score indicative of a likelihood of complications during pregnancy of the subject based on a comparison between the future GWG value and a target GWG.

A pregnancy risk score may be used to inform clinical decision making and thus may be used to improve an outcome of the pregnancy of the subject. Specifically, such information may be used to appropriately allocate resources, inform procedures, and inform recommendations for the subject.

In addition, the method may further comprise processing the data series, with a weight source prediction model, to determine a weight of the fetus, a subject hydration value and a fluid retention value.

In other words, the data series having weight values measured at least twice per day can be used to determine other relevant GWG metrics such as weight of the fetus, a subject hydration value and a fluid retention value.

According to other examples in accordance with an aspect of the invention, there is provided a computer program comprising computer program code means adapted, when said computer program is run on a computer, to implement any disclosed method for predicting future gestational weight gain of a subject.

According to another example in accordance with a further aspect of the invention, there is provided a system for predicting future GWG of a subject, comprising:
an interface configured to obtain a data series of weight values describing weight of the subject at least twice per day during an early stage of pregnancy of the subject; and
a processor configured to:
   process the data series, with a GWG prediction model, to determine a historic GWG curve of the subject describing GWG of the subject over the early stage of pregnancy; and
   predict a future GWG value of the subject based on the historic GWG curve.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 presents a flow diagram of a method for predicting future gestational weight gain (GWG) of a subject according to an embodiment of the invention;
Fig. 2 presents a block diagram of a system for predicting future GWG of a subject according to a further aspect of the invention; and
Fig. 3 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should be understood that the Figures are merely schematic and are not drawn to scale. It should also be understood that the same reference numerals are used throughout the Figures to indicate the same or similar parts.

Disclosed concepts aim to provide schemes, solutions, concepts, designs, methods, and systems pertaining to improved prediction of gestational weight gain (GWG). Specifically, a data series describing weight of the pregnant subject at least twice per day (e.g., after waking up, and before going to sleep) during an early stage of pregnancy (e.g., during the first 30 days of pregnancy, or during the first trimester) is used to determine a historic GWG curve of the subject. As energy balance in pregnancy is defined as energy intake equal to energy expenditure plus dynamic energy storage, a specific GWG prediction model is used to generate the historic GWG curve, rather than a generic weight gain model. From the historic GWG curve, a future GWG value of the subject may be predicted. This future GWG value may be used to assess a pregnancy risk score, and/or to generate lifestyle recommendations for the subject.

In general, maintaining body weight is difficult due to easy access to high caloric food, as well as an increasing trend toward sedentary lifestyles. When a person's calorie intake exceeds calorie expenditure, their body weight increases. Maintaining a target body weight may be even harder for specific demographics, such as the elderly (where signalling of hunger and satiety may be inadequate), and pregnant women (where it is unclear what weight gain is attributable to fat accumulation or foetal and placental growth). The issue of maintaining a healthy body weight is a pressing societal challenge and an unmet consumer need.

Calorie intake tends to occur intermittently and rather regularly. A daily routine can be characterized by food intake events such as breakfast, lunch, snacking and dinner. Similarly, opportunities to expend calories occur according to the daily routine. Active commuting (e.g. walking, cycling, etc.), physical exercise, occupational tasks, house chores, and sedentary moments tend to be highly repetitive on a daily and weekly basis. Calorie expenditure due to basal metabolic functions occurs continuously throughout the day as dependent on muscle mass, homeostatic processes, and digestion. With such repetitive behaviours and predictable mechanisms, maintaining a balance between calorie intake and calorie expenditure should be a feasible target for most healthy individuals. Nevertheless, reversing the effect of any weight gain or loss can be particularly difficult, often requiring long-term effort.

As an added layer of complexity, energy intake during pregnancy must match the demands of resting metabolism, physical activity, and tissue growth to enable a healthy and successful pregnancy. Energy balance in pregnancy is defined as energy intake equal to energy expenditure plus energy storage. A detailed understanding of these components and their changes throughout gestation should inform energy intake recommendations, so as to minimize the risk of poor pregnancy outcomes.

For example, inadequate energy intake during pregnancy may result in complications such as inadequate growth of the fetus, early delivery etc. Excessive energy intake during pregnancy may result in other issues, such as gestational diabetes and pre-eclampsia.

Energy expenditure is the sum of resting and physical activity-related expenditure. Resting metabolic rate increases during pregnancy as a result of increased body mass, pregnancy-associated physiological changes (e.g., changed cardiac output), and the growing fetus. Physical activity is extremely variable between pregnant individuals and may change over the course of pregnancy. Indeed, movement of pregnant individuals may be hampered by the pregnancy. The requirement for energy storage depends on maternal pre-gravid body size.

A target level of fat accumulation during pregnancy depends on the body shape/size of the pregnant individual prior to pregnancy. Those with low body weight require more fat mass accumulation than those who are overweight. That is, those with a high body weight need to accumulate less fat mass, or no fat mass at all. Given the high energy density of fat mass, these differences greatly impact energy intake requirements for a healthy pregnancy. In contrast, the energy stored in foetal and placental tissues is comparable between all pregnant individuals (but of course depends on the size of the fetus), and so has a relatively small impact on variability of energy intake requirements between women. Different prediction equations have been developed to quantify energy intake requirements.

Embodiments of the invention account for the above to enable an accurate and early prediction of future GWG, which can be used to generate personalized risk-level prediction in pregnancy. This enables early intervention and may further form the basis for improving adherence to a recommended lifestyle behavioural change during the pregnancy.

Prediction models of GWG are mostly based on regression models that use historical weight measurements during early part of the pregnancy (i.e. the first 100 days or first trimester). However, in the first 100 days, daily or even weekly weight change of the subject is so small that models are very sensitive to small inaccuracies in the measurements. These small inaccuracies may arise simply due to normal fluctuations of body weight.

By way of explanation, it has been noted that weight loss during sleep correlates with total energy intake. In addition, weight loss during sleep also correlates with individual nutritional intake, such as carbohydrate intake, fat intake, and protein intake. In other words, a person with a greater weight loss rate during the night/during sleep generally will have consumed a larger number of calories, carbohydrates, proteins and fats than a person with a lower weight loss rate. This may be explained by a variation in the resting metabolic rate of the individual consuming a greater number of calories, fats, carbohydrates or proteins.

That is, calorie/energy intake and calorie/energy expenditure can be derived from cumulative weight data measured more than once a day, for example at least once before sleep and once after sleep. An accuracy of the intake and expenditure may be improved by measuring the weight of the subject more frequently, for example once an hour, or even continuously. The automatically collected body weight information can be converted to a record of user activity and effectively guide a user towards management of body weight. Of course, during pregnancy the energy balance is defined as energy intake equal to energy expenditure plus energy storage. As energy storage requirements are dynamic during pregnancy (as well as energy expenditure due to fluctuations in resting metabolic rate, changes in exercise habits, etc.), this is a complex task for pregnant individuals. Accordingly, the invention provides a GWG prediction model which accounts for the gestational age of the subject when processing the data series of weight values, thus taking into account these possible variations during pregnancy. As a result an accurate GWG curve may be obtained from the data measured at least twice per day (preferably measured just before sleep and after just after waking).

In other words, disclosed embodiments provide early GWG predictions based on a combination of (i) a data series of weight values obtained early in pregnancy (e.g., within the first 30/100 days, or even in pre-pregnancy) measured frequently (specifically at least twice per day); and (ii) gestational age of the subject associated with the historical weight data.

Furthermore weight losses between 2 measurements can be interpreted to provide information relating to nutritional intake. Specifically, accurate measurements from before and after sleeping are known to provide information on total calorie intake, and whether the intake is fat, carbohydrate or protein based. Accordingly, this information may be leveraged to improve the prediction of future GWG, as well as to inform recommendations for improved pregnancy outcomes.

An embodiment of the proposed invention comprises some or all of the following steps:
(i) Measure the weight or (micro-) weight changes of the pregnant subject more than once per day. Typically, the weight (change) will be measured before and after sleep or at least once an hour (potentially more often or even continuous). Measurements are taken within the first trimester (although can also measure later in the pregnancy also);
(ii) Process the time-varying data series of weight values to remove noise and correct for movement, temperature, water loss, and resting metabolic rate;
(iii) Calculate the present dynamic calorie intake, nutritional intake, calorie expenditure, and calorie storage of the subject based on the measured data series, taking into account the gestational age of the subject/pregnancy condition. To perform this step, it is important to differentiate between weight increase of the subject due to fetus growth from the weight increase of the subject due to food and fluid intake which indicates the mother's weight change and thereby indirectly the calorie intake;
(iv) Determine the GWG curve up to the current/latest weight measurement in the data series, by processing the data series and associated gestational age with a GWG prediction model;
(v) Predict the GWG at later time points, (e.g., in the 2nd and/or 3rd trimester) using the GWG curve;
(vi) Compare and classify the predicted future GWG to recommended GWG based on applicable guidelines (such as IOM 2009 guideline) to assess the risk or risk level of pregnancy. For example, the comparison may be used to determine a chance of the subject developing preeclampsia, gestational diabetes, etc.; and
(vii) Calculate a recommended change to the dynamic calorie intake and calorie expenditure, based on measured present dynamic calorie and nutritional intake and calorie expenditure, deviation of predicted future GWG to a recommended/target GWG, and dynamic energy requirements during pregnancy. For example, the recommended change may be related to an amount of physical activity, and/or nature of physical activity. Optionally, a gait stability of the subject is measured, using a body-worn accelerometer, which may be used to adapt the recommended physical activity. Furthermore, the body-worn accelerometer may be used to measure activity behaviour of the subject, and thus adjust the recommendation for more or less physical activity.

As well as the above, methods may also facilitate prediction of the growth of the fetus (e.g., a weight of the baby) from the data series of weight values. Similarly, body hydration and fluid retention may be determined from the data series of weight values. Optionally, other data can be obtained such as physical activity, heart rate, blood pressure, ultrasound or impedance to determine fluid content (i.e., to distinguish baby weight and surrounding fluids so as to determine fluid content). In some embodiments, an accelerometer at the waist/belly of the subject may sense fetus growth by tilt increment.

Further, the calculated recommended calorie intake may be linked to a nutritional recommendation unit that takes into account the target calorie and the preferred types of food of the mother and recommends the amount of food.

To reiterate, weight losses between two weight value measurements can be used to provide information related to nutritional intake during pregnancy. Generally, the weight loss during the night can be inferred as weight being used to maintain the subject's body and in the form of water and heat loss. As such all energy expenditure results in loss of weight. If not all energy is expended it may built up as stored energy in the form of fat.

However, it has been realised that for pregnant individuals this mechanism is different. In addition to the usual energy expenditures which lead to weight loss and storage of additional energy in fat, the pregnant subject also uses energy (and other nutrients) to develop the fetus. These proteins, fluids, minerals and additional fats used to develop the baby are directly consumed through the baby's growth (and own metabolic processes) or (temporally) stored with the mother.

As such, whilst accurate measurements taken from before and after sleeping at the beginning of the pregnancy are able to provide information on total calory intake and to assess whether the intake is fat, carbohydrate or protein based, this will become more complex as the pregnancy develops (i.e., with greater gestational age). Specifically, the nutritional intake of the subject may be underestimated. In order to account for this, additional terms relating to known usage of energy to develop the baby at different stages of development need to be added to the model as an energy usage which does not lead to a weight loss. Of course, this will all scale with gestational age of the subject. In other words, such energy usage will scale with the weight of the developing baby, whereby a knowledge of the weight of the baby (from e.g. ultrasound images) will improve the accuracy of the models.

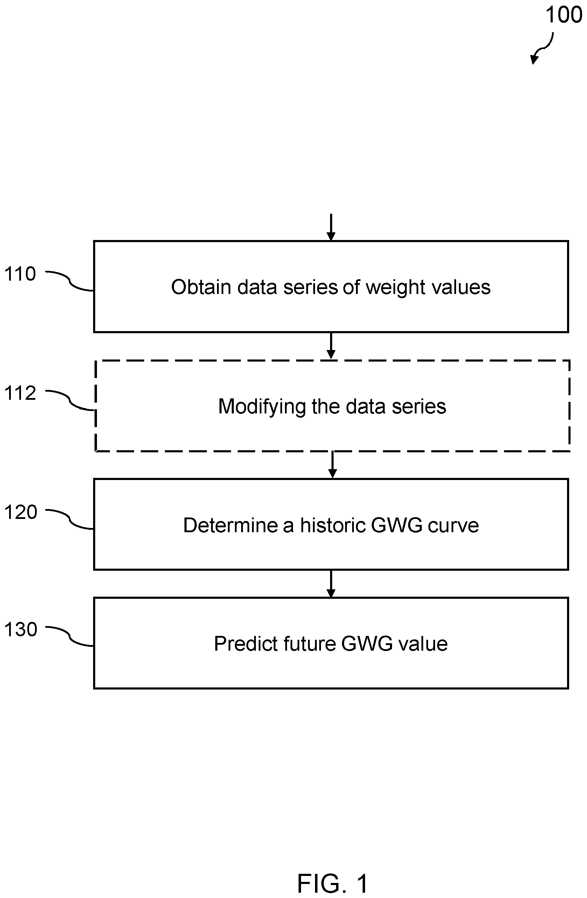

Fig. 1 presents a flow diagram of a method for predicting future GWG of a subject according to an embodiment of the invention.

GWG is a natural response to accommodate the growing fetus. Components of GWG include the body composition (fat, lean mass), the weight of the fetus, placenta, and amniotic fluid. Nevertheless, a GWG that is too high or two low can contribute to short-term and long-term health complications. Therefore, it is desirable to predict future GWG such that preventative or mitigating actions may be taken at an earlier stage (responsive to predicting that the future GWG falls outside of a recommended range).

This is achieved by disclosed embodiments by leveraging micro-weighing data, or weight data measured at least twice per day, during an early stage of pregnancy (e.g., the first 100 days, or first trimester, of pregnancy). By taking at least two weight readings a day, calorie intake and expenditure for each day may be taken into account. As a result, an accurate understanding of the historic GWG of the subject (i.e. the historic GWG curve) may be achieved. In addition, by processing the data series with a GWG prediction model that also considers the gestational age of the subject (and therefore the variability/dynamic nature of the calorie expenditure and storage during pregnancy), an accurate (i.e., close to ground-truth) GWG curve describing the historic GWG of the subject may be determined. From this determined GWG curve, future GWG value(s) may be generated.

In step 110, a data series of weight values is obtained. The data series of weight values describes the weight of the subject at least twice per day during an early stage of pregnancy of the subject and a respective gestational age of the subject. That is, the data series comprises a plurality of weight values, which describe weight of the subject, and respective gestational age of the subject when those weight values were taken.

In particular, the data series of weight values may describe the weight of the subject at least each day before sleep and after sleep. If possible, the data series of weight values may describe the weight of the subject at least once per hour. These instances (i.e., when the weight of the subject is measured at least twice per day) may be described as micro-weighing of the subject, which enables a model processing the data series to account for normal fluctuations in weight of the subject.

Furthermore, the data series of weight values may further describe the weight of the subject at least twice per day during a time period preceding pregnancy. Thus, changes in weight attributable to pregnancy and those attributable to other sources may be more straightforward to differentiate.

The data series may be obtained from a database, or may be directly measured from a connected device, such as a dedicated set of scales.

In (optional) step 112, the data series is modified/augmented/corrected based on subject context information. The subject context information may contain any information related to variations in the measurement of the weight of the subject, such that the weight values may be normalised. For example, the subject context information may comprise at least one of movement, temperature, water loss, or resting metabolic rate of the subject during the early stage of pregnancy of the subject.

For example, if the subject has experienced a large amount of water loss, the weight value at that time may be adjusted to be greater.

In step 120, the data series is processed with a GWG prediction model. Accordingly, a historic GWG curve of the subject is determined/generated, which describes GWG of the subject over the early stage of pregnancy.

The GWG prediction model may be a machine learning model trained using a training algorithm using a training set. The training set may comprise a set of historic data series of weight values of a plurality of subjects and respective gestational ages, and an associated known GWG curve corresponding to each data series. Accordingly, the GWG prediction model may be trained to output a GWG curve responsive to receiving a data series of weight values.

Alternatively, the GWG prediction model could be any model adapted to compensate for the dynamic calorie expenditure and storage requirements during pregnancy, which depend on the gestational age of the subject. Thus, the GWG prediction model is able to generate a GWG curve that is reflective of the ground-truth GWG of the subject during the early stage of the pregnancy. In particular, the GWG prediction model may comprise a regression analysis model such as a maximum a-posteriori regression analysis model or gaussian processes regression analysis model.

In step 130, a future GWG value of the subject is predicted based on the historic GWG curve. For example, a prediction of the GWG value of the subject at a point during the second or third trimester is generated. The predicted future GWG value may be a numerical value (i.e., a specific weight), or may be a categorical value (e.g., too high, too low, etc).

The predicted future GWG value may then be adjusted based on weight-related physiological data. That is, weight-related physiological data describing at least one physiological parameter of the subject is obtained. The predicted future GWG value is then adjusted based on the obtained weight-related physiological data.

The at least one physiological parameter may comprise at least one of a pre-pregnancy weight, a pre-pregnancy BMI, an age, a height, an ethnicity, a stress value a pre-pregnancy lifestyle parameter value, or a pregnancy lifestyle parameter value. Nevertheless, any other physiological parameter that has relevance to weight and GWG of the subject may be considered.

Of course, the predicted future GWG value may then be used for various purposes. The future GWG value may simply be output to the subject and/or a medical professional for further analysis. Additionally, a pregnancy risk score indicative of a likelihood of complications during pregnancy of the subject may be generated or augmented/adjusted based on a comparison between the future GWG value and a target GWG (i.e., an ideal GWG of the subject according to IOM 2009 guidelines).

Other embodiments of the invention may be based on a further step of generating at least one of current calorie intake data, current calorie expenditure data, current calorie storage data or current nutritional intake data based on the data series and a current gestational age of the subject. As stated above, these parameters may be derived from at least two weight measurements of the subject per day.

In this case, determining the historic GWG curve of the subject may further comprise processing the at least one of the current calorie intake data, current calorie expenditure data, current calorie storage data or current nutritional intake data with the GWG prediction model. Indeed, the determined/generated historic GWG curve may be more accurate if calorie intake, expenditure and storage data, as well as nutritional data, is considered.

Furthermore, a recommendation describing a recommended change to a lifestyle of the subject may be generated based on the future GWG value, and at least one of the current calorie intake data, current calorie expenditure data, current calorie storage data or current nutritional intake data. Indeed, these factors will directly impact the future GWG value. Thus, adjusting one or more of these factors may enable an adjustment of the true future GWG value to be closer to a target/ideal GWG value.

The recommendation may comprise a calorie intake recommendation, a nutritional intake recommendation, or an exercise recommendation. When the recommendation comprises an exercise recommendation, this may be adjusted based on gait information describing a gait stability of the subject (so that the exercise recommendation is realistic and safe for the abilities of the subject).

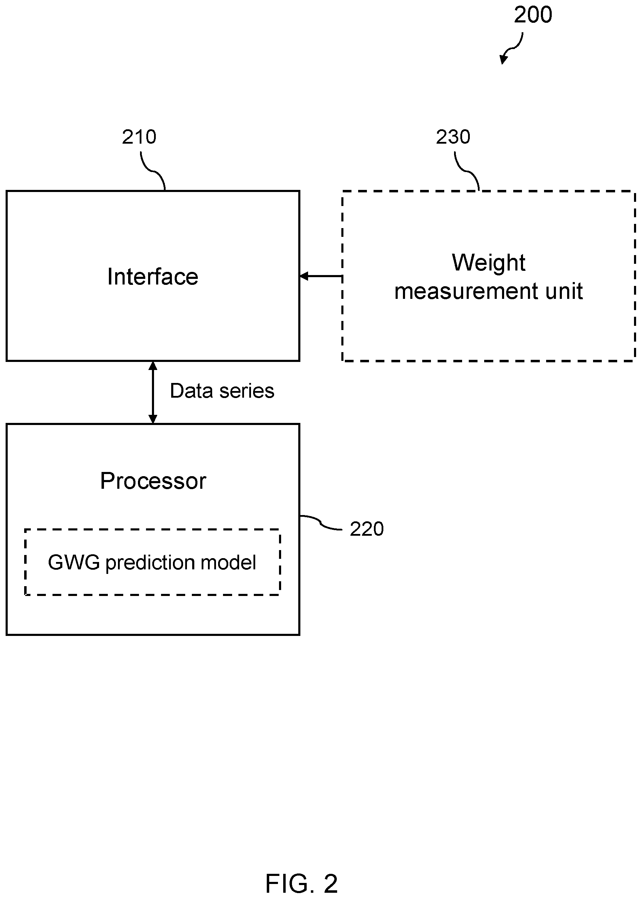

Fig. 2 presents a block diagram of a system 200 for predicting future GWG of a subject. The system 200 may be implement any method as described above. Specifically, the system comprises an interface 210, and a processor 220. The system 200 may also comprise a weight measurement unit 230.

Specifically, the interface 210 configured to obtain a data series of weight values describing weight of the subject at least twice per day during an early stage of pregnancy of the subject and a respective gestational age of the subject.

The interface 210 may obtain the data series from the weight measurement unit 230, which is configured to measure the weight of the subject.

The processor 220 is configured to process the data series, with a GWG prediction model, to determine a historic GWG curve of the subject describing GWG of the subject over the early stage of pregnancy.

Furthermore, the processor 220 is configured to predict a future GWG value of the subject based on the historic GWG curve.

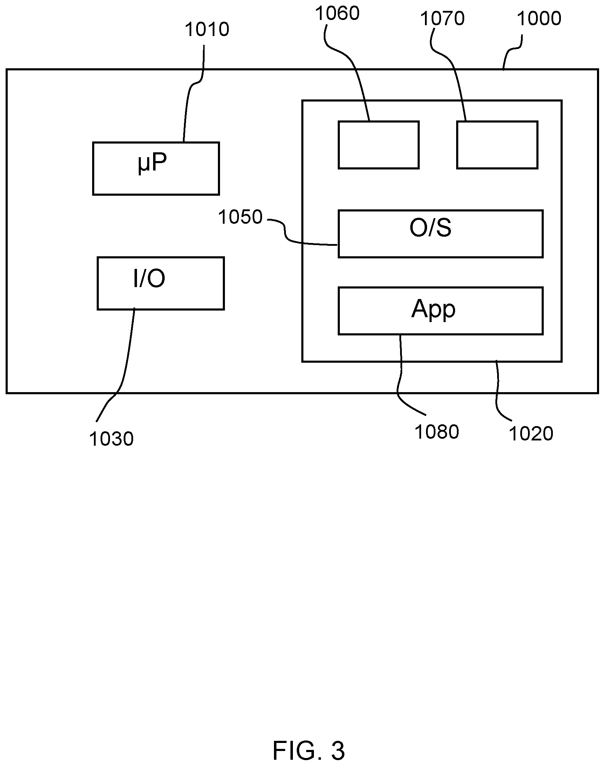

Fig. 3 illustrates an example of a computer 1000 within which one or more parts of an embodiment may be employed. Various operations discussed above may utilize the capabilities of the computer 1000. For example, one or more parts of a system for acquiring an input from a user to control an interface may be incorporated in any element, module, application, and/or component discussed herein. In this regard, it is to be understood that system functional blocks can run on a single computer or may be distributed over several computers and locations (e.g. connected via internet). Further, the computer 1000 may be implemented in a networked/distributed system, such as where computation occurs in the cloud.

The computer 1000 includes, but is not limited to, smart phones, PCs, workstations, laptops, PDAs, palm devices, servers, storages, patient monitors and the like. In many cases, the disclosed systems and methods may be implemented in a patient monitor, such as a bed side monitor. Such monitors may be used in, for example, the intensive care unit (ICU), the operating room (OR), or post-anaesthesia care unit (PACU).

Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), a graphics processing unit (GPU), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), solid state drive (SSD), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. The application 1080 for implementing the exemplary and comparable embodiments can be made applicable on all (general purpose) operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written in an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, functional programming and the like.

The I/O devices 1030 may include input devices such as, for example but not limited to, a mouse, keyboard, scanner, microphone, camera, touch-screen, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods described in relation to Fig. 1, and the system described in relation to Fig. 2, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, EEPROM, and SSD, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram of Fig. 2 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method for predicting future gestational weight gain, GWG, of a subject, comprising:
obtaining (110) a data series of weight values describing weight of the subject at least twice per day during an early stage of pregnancy of the subject and a respective gestational age of the subject;
processing the data series, with a GWG prediction model, to determine (120) a historic GWG curve of the subject describing GWG of the subject over the early stage of pregnancy; and
predicting (130) a future GWG value of the subject based on the historic GWG curve.

2. The method of claim 1, wherein the data series of weight values describe the weight of the subject at least each day before sleep and after sleep.

3. The method of claim 1 or 2, wherein the data series of weight values describe the weight of the subject at least once per hour.

4. The method of any of claims 1-3, wherein the data series of weight values further describe the weight of the subject at least twice per day during a time period preceding pregnancy.

5. The method of any of claims 1-4 further comprising generating at least one of current calorie intake data, current calorie expenditure data, current calorie storage data or current nutritional intake data based on the data series and a current gestational age of the subject.

6. The method of claim 5, wherein determining (120) the historic GWG curve of the subject further comprises processing the at least one of the current calorie intake data, current calorie expenditure data, current calorie storage data or current nutritional intake data with the GWG prediction model.

7. The method of claim 5 or 6, further comprising generating a recommendation describing a recommended change to a lifestyle of the subject based on the future GWG value, and at least one of the current calorie intake data, current calorie expenditure data, current calorie storage data or current nutritional intake data, and
optionally wherein the recommendation comprises a calorie intake recommendation, a nutritional intake recommendation, or an exercise recommendation.

8. The method of claim 7, wherein the recommendation comprises an exercise recommendation, further comprising adjusting the exercise recommendation based on gait information describing a gait stability of the subject.

9. The method of any of claims 1-8, further comprising modifying (112) the data series based on subject context information describing at least one of movement, temperature, water loss, cardiovascular activity, or resting metabolic rate of the subject during the early stage of pregnancy of the subject.

10. The method of any of claims 1-9, further comprising:
obtaining weight-related physiological data describing at least one physiological parameter of the subject; and
adjusting the predicted future GWG value based on the obtained weight-related physiological data, and
optionally wherein the at least one physiological parameter comprises at least one of a pre-pregnancy weight, a pre-pregnancy BMI, an age, a height, an ethnicity, a stress value a pre-pregnancy lifestyle parameter value, or a pregnancy lifestyle parameter value.

11. The method of any of claims 1-10, wherein processing the data series to determine (120) the historic GWG curve comprises processing the data series with a regression analysis model, and wherein predicting (130) the future GWG value of the subject comprises extrapolating the historic GWG curve.

12. The method of any of claims 1-11, further comprising generating a pregnancy risk score indicative of a likelihood of complications during pregnancy of the subject based on a comparison between the future GWG value and a target GWG.

13. The method of any of claims 1-12, further comprising processing the data series, with a weight source prediction model, to determine a weight of the fetus, a subject hydration value and a fluid retention value.

14. A computer program comprising computer program code means adapted, when said computer program is run on a computer, to implement the method of any of claims 1-13.

15. A system for predicting future gestational weight gain, GWG, of a subject, comprising:
an interface (210) configured to obtain a data series of weight values describing weight of the subject at least twice per day during an early stage of pregnancy of the subject and a respective gestational age of the subject; and
a processor (220) configured to:
process the data series, with a GWG prediction model, to determine a historic GWG curve of the subject describing GWG of the subject over the early stage of pregnancy; and
predict a future GWG value of the subject based on the historic GWG curve.
